# EUROPÄISCHE PATENTSCHRIFT

(11) **EP 1 527 791 B1**
(45) Veröffentlichungstag und Bekanntmachung des Hinweises auf die Patenterteilung: **26.09.2007**
(21) Anmeldenummer: 05002552.7
(22) Anmeldetag: 26.02.1997
(51) Int. Cl.: A61M 1/00

(54) **Einrichtung zum Absaugen von Flüssigkeiten**
Fluid suction device
Dispositif de succion de fluides

(43) Veröffentlichungstag der Anmeldung: 04.05.2005
(62) Teilanmeldung aus: 97103068.9
(73) Patentinhaber: Medela Holding AG, 6340 Baar (CH)
(72) Erfinder: Greter, Andy, 6312 Steinhausen (CH)
(74) Vertreter: Clerc, Natalia

(56) Entgegenhaltungen:
- EP-A- 0 378 296
- WO-A-94/14045
- US-A- 3 680 560
- US-A- 4 306 557
- US-A- 4 821 896

## Beschreibung

Die vorliegende Erfindung betrifft einen Einwegbeutel zum Absaugen von Flüssigkeiten, insbesondere von Körperflüssigkeiten im Rahmen medizinischer Behandlungen, gemäss Oberbegriff des Patentanspruchs 1.

Im medizinischen Bereich sind für das Absaugen von Körperflüssigkeiten heute die verschiedensten Systeme bzw. Methoden bekannt. In all jenen Fällen, wo die Körperflüssigkeiten durch Anwendung von Unterdruck abzusaugen und aufzufangen sind, kann dies z.B. dadurch geschehen, dass ein Auffangbehälter aus starrem Material mit einem Deckel versehen wird, in welchen einerseits die Absaugleitung führt und andererseits ein Anschluss zum Aufbau eines Unterdruckes vorgesehen ist, wobei dieser Anschluss zu einer Saugquelle (Saugpumpe) führt. Das Problem bei diesem System liegt darin, dass zur Entsorgung der aufgefangenen Flüssigkeiten der Behälter als Ganzes zu transportieren ist und nach dem Entleeren des Inhaltes einer sorgfältigen Reinigung zu unterziehen ist. Dieses System entspricht nach neuesten Erkenntnissen in gewissen Fällen den Anforderungen an die Hygiene nicht mehr. Um dieses Problem zu lösen, wurde vorgeschlagen, im Innern des starren Behälters einen Einwegbeutel unterzubringen, welcher allein zum Auffangen der abzusaugenden Flüssigkeiten dient. Ein solcherart mit Flüssigkeit gefüllter Beutel kann zur Entsorgung nach Abnehmen des Behälterdeckels entfernt und der eigentlichen Entsorgung zugeführt werden, während der starre Behälter in der Regel bereits mit einem neuen Beutel ausgerüstet werden kann. Das Problem bei diesem System liegt darin, dass zwei verschiedene Unterdruck-Leitungen (Vakuum-Leitungen) erforderlich sind, nämlich die eine, um den Beutel aufzuweiten und diesen gegen die Behälter-Innenwand anzulegen, und ein zweiter Anschluss, welcher ins Beutelinnere führt, um nach dessen Aufweitung im Beutelinnern den erforderlichen Unterdruck zu erzeugen. Dabei ist immer darauf zu achten, dass der Unterdruck im den Beutel umgebenden Raum höher sein muss als jener im Innern des Beutels, da sonst die Gefahr besteht, dass der Beutel wieder zusammenklappt und eine weitere Füllung nicht mehr möglich ist. Ganz abgesehen davon, ist eine solche Konstruktion aufwendig und somit teuer.

Die WO 94/14045 offenbart einen Behälter mit einem Einwegbeutel zur Aufnahme von abzusaugender Körperflüssigkeit. Der Beutel weist eine Kopfplatte aus einem starren Material auf. Diese Kopfplatte bildet den Deckel des Behälters und verfügt über entsprechende Öffnungen zum Anschluss an die Saugpumpe und an den Drainageschlauch. Ferner weist der untere Bereich des Beutels ein ausbrechbar gestaltetes Ventil auf.

Aufgabe der vorliegenden Erfindung war es somit, einen Einwegbeutel zu schaffen, mit welchem bei einfachster Konstruktion das Auffangen von Flüssigkeiten problemlos möglich ist.

Diese Aufgabe wird erfindungsgemäss durch einen Einwegbeutel mit den Merkmalen gemäss Anspruch 1 gelöst.

Dank der erfindungsgemässen Konstruktion wird es mit einer einzigen Unterdruckleitung möglich, zuerst den flexiblen Behälter im Innern des luftdicht abgeschlossenen Auffangbehälters aufzuweiten und in dieser Stellung zu halten und erst nach dem Aufweiten die Saugwirkung ins Innere des Beutels zu verlegen. Beim aufgeweiteten Beutel, welcher durch den angelegten Unterdruck immer in diesem erforderlichen Zustand gehalten wird, sorgt die gasdurchlässige Membran dafür, dass durch diese keine Flüssigkeit austreten kann (Sperrmembran), jedoch Luft zwecks Erzeugung des erforderten Unterdruckes abgesaugt werden kann. Durch diese einfache Massnahme, welche sich durch das starre Kopfteil am Beutel realisieren lässt, entsteht eine ausserordentlich einfach aufgebaute Einrichtung. Das ausbrechbare Element verschliesst im Normalfall eine Durchgangsöffnung. Diese Öffnung kann beim Entleeren des Beutels als Entlüftung dienen.

Besondere Ausführungsformen sind in den abhängigen Ansprüchen definiert.

Es sei nochmals wiederholt, dass beim Einsatz von Einwegbeuteln dafür Sorge getragen werden muss, dass beim Eintritt der Saugwirkung die Beutelwände nicht einfach zusammenklappen, sondern dass der Beutel zuerst in Richtung der Wand des starren Auffangbehälters aufgeweitet bzw. gegen die Behälterwände angelegt werden muss. Das war bisher nur unter Einrichtung von zwei verschiedenen Vakuumleitungen möglich, wobei die eine zum Aufweiten des Beutels diente, die andere zum Erzeugen des Vakuums im Beutelinnem. Nachteilig an diesem System war einerseits der komplexe Aufbau, und zudem konnte die Wirkung nicht immer gewährleistet werden, ohne die Einrichtung nochmals zu komplizieren.

Die Erfindung wird anschliessend anhand eines in der Zeichnung dargestellten Ausführungsbeispiels noch etwas näher erläutert. Es zeigen:
- Fig. 1: Einen Vertikalschnitt durch einen starren Auffangbehälter, in welchen ein flexibler Einwegbeutel mit starrem Kopfteil eingesetzt ist;
- Fig. 2: rein schematisch eine Ansicht von unten auf die Unterseite des Behälter-Deckels mit eingesetztem Einwegbeutel und
- Fig. 3: eine perspektivische Ansicht des starren Kopfteils eines Einwegbeutels gemäss Fig. 1.

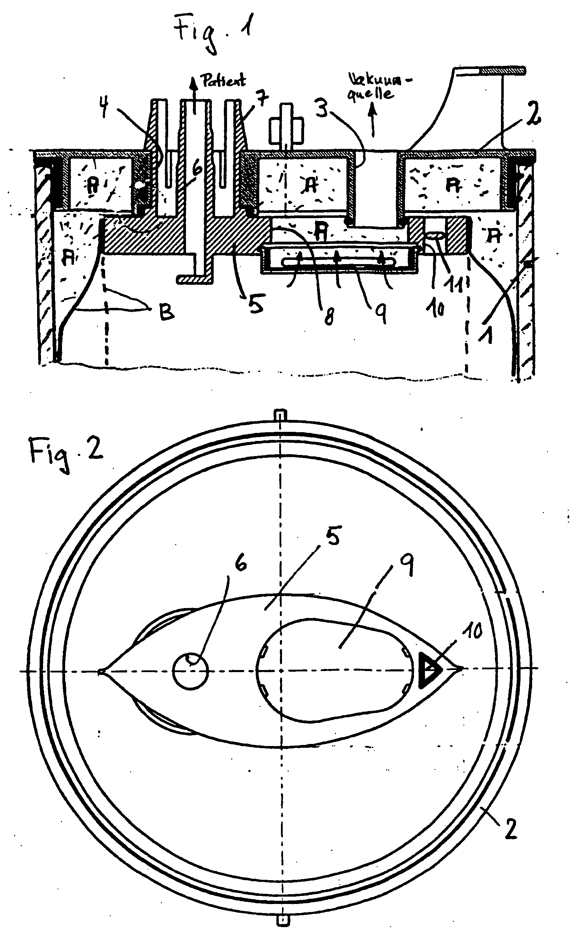

Figur 1 der Zeichnung zeigt rein schematisch eine Einrichtung mit einem Einwegbeutel nach der Erfindung. Dabei ist auf einen starren Behälter 1 ein Deckel 2 so aufgesetzt, dass ein luftdichter Verschluss entsteht. Dies wird durch entsprechende Dichtungen zwischen Behälter 1 und Deckel 2 realisiert. Im Deckel 2 selbst ist eine Anschlussöffnung 3 für den Anschluss einer Verbindungsleitung zu einer Saugquelle (Saugpumpe, nicht dargestellt) vorgesehen, in welche die Verbindungsleitung ebenfalls luftdicht einsteckbar oder anderweitig anschliessbar ist. Der Behälterdeckel 2 weist ferner eine Anschlussöffnung 4 zum Anbringen einer zum Patienten führenden Absaugleitung auf (nicht dargestellt).

Wie die Zeichnung zeigt, ist von der Unterseite des Behälterdeckels her ein starres Kopfteil 5 eines flexiblen Einwegbeutels B befestigt. Das starre Kopfteil 5 besteht aus einer schiffchenförmigen Platte, von welcher einerseits ein Anschlussnippel 6 durch die Behälterdeckel-Öffnung 4 nach oben führt und welche von federnden Rastklinken 7 umgeben ist. Der Nippel 6 dient zum Aufstecken der zum Patienten führenden Absaugleitung. Zwischen der Kopfplatte 5 des Beutels B und dem Behälterdeckel 2 ist eine Dichtung vorgesehen, um die Verbindung luftdicht zu gestalten. Die Rastklinken 7 lassen sich durch die Deckelöffnung 4 hindurchführen und spreizen dann in die Halteposition aus, wie dies aus Fig. 1 hervorgeht. Damit ist die starre Platte 5 des Einwegbeutels fest auf der Unterseite des Deckels angebracht. Bei angeschlossener Absaugleitung und Verbindungsleitung zur Saugquelle ist klar ersichtlich, dass der Innenraum des Behälters 1 gegenüber der Umgebung völlig abgedichtet ist.

In der Kopfplatte 5 des Beutels ist eine weitere Öffnung 8 vorgesehen, in welcher eine Membran 9 festgehalten ist. Diese Membran 9 ist bezüglich Flüssigkeiten undurchlässig, jedoch unter Überwindung eines geringen Widerstandes für Gas bzw. Luft durchlässig.

In der Kopfplatte 5 des Beutels B ist eine weitere Durchgangsöffnung 10 vorgesehen, welche im Normalfall durch ein ausbrechbares Element 11 verschlossen ist. Diese Öffnung könnte dazu dienen, bei Entleerung eines Beutels B als Entlüftung zu dienen, um das Ausströmen der aufgefangenen Flüssigkeit durch den Nippel 6 hindurch zu erleichtern, falls dies notwendig wäre. In der Regel wird allerdings eine Entleerung durch Druck auf die Beutelwand ausreichen.

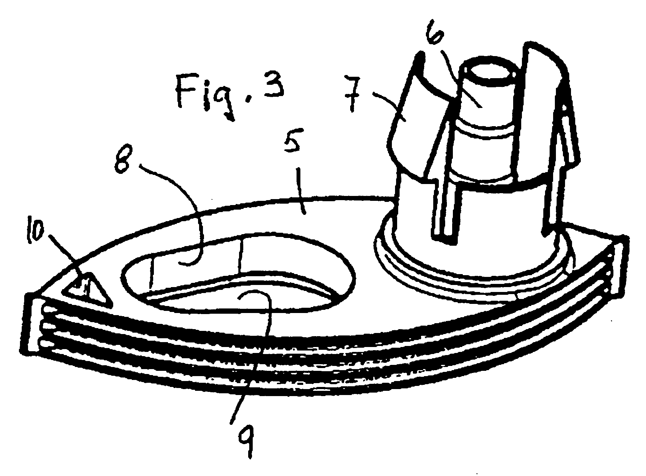

Zur allgemeinen Illustration zeigt Fig. 3 noch eine perspektivische Darstellung des starren Kopfteils 5 eines Einwegbeutels B.

Wenn nun mit auf der Unterseite des Deckels 2 angebrachtem Beutel (mit im Deckel festgehaltener bzw. festgeklemmter Kopfplatte 5 des Beutels B, angeschlossener, zur Vakuumquelle führender Leitung und angeschlossener Patientenleitung) die Vakuumquelle in Betrieb genommen wird, d.h. durch den Anschluss hindurch eine Saugwirkung erzeugt wird, wird zuerst am oberen Ende des Behälters, in den Räumen A die Luft evakuiert und damit der Beutel B mit seinen Seitenwänden in Richtung zu den Behälterwänden 1 aufgeweitet bzw. an diese angelegt, danach wird auch die Luft durch die Membrane 9 hindurch im Beutelinnern abgesaugt und dabei ein Unterdruck erzeugt, welcher wiederum dafür sorgt, dass die abzusaugende Flüssigkeit über den Patientenanschluss ins Innere des Beutels B gelangt.

Durch die einfache Konstruktion, insbesondere des starren Kopfteils 5 des Einwegbeutels, ist eine Einrichtung geschaffen, welche mit einem minimalen Konstruktionsaufwand das Absaugen von Flüssigkeiten in einen Einwegbeutel ermöglicht. Gleichzeitig eignet sich diese Einrichtung auch für den normalen Einsatz, d.h. das Absaugen von Flüssigkeiten direkt in den starren Behälter 1. Dazu kann entweder derselbe Deckel 2 verwendet werden oder aber ein separater Deckel vorgesehen sein, welcher einen Anschluss für eine Absaugleitung und einen Anschluss für eine Vakuumquelle aufweist.

## Patentansprüche

1. Einwegbeutel zur Aufnahme von abzusaugender Flüssigkeit, wobei er mit einer Kopfplatte (5) aus starrem Material ausgerüstet ist, wobei einerseits ein nach oben abstehender Anschluss für eine Absaugleitung vorgesehen ist, welcher Anschluss gleichzeitig als Befestigungsvorrichtung ausgebildet ist, um den Beutel von der Unterseite her an einem Deckel (2) eines starren Auffangbehälters (1) zu befestigen, und andererseits eine mit einer flüssigkeitssperrenden, aber gasdurchlässigen Membran (9) verschlossene Öffnung (8) vorgesehen ist, durch welche im Beutelinnern ein Unterdruck erzeugbar ist, wobei der Einwegbeutel ein ausbrechbares Element (11) aufweist, welches eine Durchgangsöffnung (10) verschliesst, **dadurch gekennzeichnet, dass**
die Durchgangsöffnung (10) im Kopfteil (5) des Einwegbeutels vorgesehen ist.

2. Einwegbeutel nach Anspruch 1, **dadurch gekennzeichnet, dass** die Durchgangsöffnung (10) ein eine Entlüftungsöffnung bildender Durchgang ist.

3. Einwegbeutel nach Anspruch 1, **dadurch gekennzeichnet, dass** der Anschluss der Absaugleitung am Kopfteil (5) des Beutels als Anschlussnippel (6) ausgebildet ist, welcher von konzentrisch dazu angeordneten Halte- und Kupplungsgliedern umgeben ist, welche ihrerseits zur Kupplung des Kopfteils (5) des Beutels mit dem Behälterdeckel dienen.

4. Einrichtung nach Anspruch 3, **dadurch gekennzeichnet, dass** die Kupplungsglieder als den Anschlussnippel (6) konzentrisch umgebende, nach oben abstehende federnde Rastklinken (7) ausgebildet sind und zwischen der Nippelaussenwand und Innenseite der Rastklinken (7) ein nach oben offener Ringraum zum Einstecken der Absaugleitung vorgesehen ist.

## Claims

1. Disposable bag for receiving fluid that is to be suctioned off, said disposable bag being equipped with a head plate (5) made of rigid material and having, on the one hand, an upwardly protruding connector for a suction line, which connector is at the same time designed as a securing device for securing the bag from the underside on a lid (2) of a rigid collecting container (1), and, on the other hand, an opening (8) which is sealed by a fluid-tight but gas-permeable membrane (9) and through which an underpressure can be generated in the inside of the bag, and the disposable bag having a breakable element (11) that seals off a through-opening (10), **characterized in that** the through-opening (10) is provided in the head part (5) of the disposable bag.

2. Disposable bag according to Claim 1, **characterized in that** the through-opening (10) is a passage forming an air vent.

3. Disposable bag according to Claim 1, **characterized in that** the connector of the suction line on the head part (5) of the bag is designed as a connector nipple (6) surrounded by holding and coupling members that are arranged concentrically with respect to it and in turn serve for coupling the head part (5) of the bag to the container lid.

4. Device according to Claim 3, **characterized in that** the coupling members are designed as upwardly protruding resilient catches (7) that concentrically surround the connector nipple (6), and, between the outside wall of the nipple and the inside of the catches (7), an annular space is provided which is open to the top and serves for insertion of the suction line.

## Revendications

1. Sachet à usage unique pour recevoir des liquides à aspirer, ce sachet étant muni d'une plaque de tête (5) en matériau rigide, un raccord pour une conduite de succion, saillant vers le haut, étant d'une part prévu, lequel est réalisé en même temps en tant que dispositif de fixation, pour fixer le sachet par le côté inférieur à un couvercle (2) d'un récipient collecteur rigide (1), et d'autre part une ouverture (8) fermée par une membrane (9) bloquant le passage de fluides mais perméable aux gaz, qui peut produire une dépression à l'intérieur du sachet, le sachet à usage unique présentant un élément cassable (11) qui ferme une ouverture de passage (10),
**caractérisé en ce que**
l'ouverture de passage (10) est prévue dans la partie de tête (5) du sachet à usage unique.

2. Sachet à usage unique selon la revendication 1, **caractérisé en ce que** l'ouverture de passage (10) est un passage formant une ouverture de désaérage.

3. Sachet à usage unique selon la revendication 1, **caractérisé en ce que** le raccord de la conduite de succion au niveau de la partie de tête (5) du sachet est réalisé sous forme de raccord fileté (6) qui est entouré par des organes de retenue et d'accouplement disposés concentriquement par rapport à lui, et qui pour leur part servent à l'accouplement de la partie de tête (5) du sachet au couvercle du récipient.

4. Dispositif selon la revendication 3, **caractérisé en ce que** les organes d'accouplement sont réalisés sous forme de cliquets (7) à ressort saillant vers le haut et entourant concentriquement le raccord fileté (6), et **en ce qu'**un espace annulaire ouvert vers le haut est prévu entre la paroi extérieure du raccord et le côté intérieur des cliquets (7) pour enfoncer la conduite de succion.
